# FASCICULE DE BREVET EUROPEEN

(11) **EP 1 281 774 B1**
(45) Date de publication et mention de la délivrance du brevet: **25.12.2013**
(21) Numéro de dépôt: 02012081.2
(22) Date de dépôt: 08.11.1995
(51) Int. Cl.: C12Q 1/68

(54) **Support pour la purification d'un ADN double brin**
Träger zur Reinigung doppelsträngiger DNA
Support for purifying a double-strand DNA

(30) Priorité: 16.12.1994 FR 9415162
(43) Date de publication de la demande: 05.02.2003
(62) Demande divisionnaire de: 95940285.0
(73) Titulaire: Aventis Pharma S.A., 92160 Antony (FR)
(72) Inventeur: Couzet, Joel, 92330 Sceaux (FR); Scherman, Daniel, 75012 Paris (FR); Wils, Pierre, 75005 Paris (FR)
(74) Mandataire: Holtz, Béatrice

(56) Documents cités:
- WO-A-92/09705
- WO-A-92/11390
- WO-A-93/00352
- US-A- 4 835 263
- ITO ET AL.: "Sequence-specific DNA purification by triplex affinity capture" PROCEEDINGS OF THE NATIONAL ACADEMY OF SCIENCES OF USA, vol. 89, pages 495-498, XP002264004 WASHINGTON US
- ZUIDEMA D ET AL: "THE ISOLATION OF DUPLEX DNA FRAGMENTS CONTAINING (DG.DC) CLUSTERS BY CHROMATOGRAPHY ON POLY(RC)-SEPHADEX" NUCLEIC ACIDS RESEARCH, OXFORD UNIVERSITY PRESS, SURREY, GB, vol. 5, no. 7, 1 juillet 1978 (1978-07-01), pages 2471-2483, XP000603141 ISSN: 0305-1048
- FLAVELL R A ET AL: "THE ISOLATION OF DUPLEX DNA CONTAINING (DA.DT) CLUSTER BY AFFINITY CHROMATOGRAPHY ON POLY(U) SEPHADEX" FEBS LETTERS, XX, XX, vol. 58, no. 1, 15 octobre 1975 (1975-10-15), pages 90-93, XP000603017 ISSN: 0014-5793
- HANVEY J C ET AL: "SITE-SPECIFIC INHIBITION OF ECORI RESTRICTION/MODIFICATION ENZYMES BY A DNA TRIPLE HELIX" NUCLEIC ACIDS RESEARCH, OXFORD UNIVERSITY PRESS, SURREY, GB, vol. 18, no. 1, 1990, pages 157-161, XP002026239 ISSN: 0305-1048
- JARRETT H W: "AFFINITY CHROMATOGRAPHY WITH NUCLEIC ACID POLYMERS" JOURNAL OF CHROMATOGRAPHY, BIOMEDICAL APPLICATIONS, ELSEVIER SCIENCE PUBLISHERS B.V. AMSTERDAM, NL, vol. 618, 1993, pages 315-339, XP002000129

## Description

La présente invention concerne un support pour la purification d'ADN, tel que décrit dans la revendication 1.

Les techniques de thérapie génique et cellulaire connaissent actuellement un développement extraordinaire. Néanmoins, ces techniques impliquent la possibilité de produire des quantités importantes d'ADN de pureté pharmaceutique. En effet, dans ces nouvelles thérapies, le médicament est souvent constitué par l'ADN même, et il est essentiel de pouvoir le fabriquer, dans des quantités adaptées, l'isoler et le purifier de manière appropriée à un usage thérapeutique chez l'homme.

Est décrit un nouveau procédé simple et particulièrement efficace pour la purification d'ADN. Il permet notamment d'obtenir des puretés particulièrement hautes, avec des rendements élevés.

Le procédé repose essentiellement sur une interaction spécifique entre une séquence insérée sur l'ADN à purifier et un oligonucléotide composé de bases naturelles ou modifiées.

Il a récemment été montré que certains oligonucléotides sont capables d'interagir spécifiquement dans le grand sillon de la double-hélice d'ADN pour former localement des triple-hélices, conduisant à une inhibition de la transcription de gènes-cible (Hélène et Toulmé, Biochim. Biophys. Acta 1049 (1990) 99). Ces oligonucléotides reconnaissent sélectivement la double-hélice d'ADN au niveau de séquences oligopurine.oligopyrimidine, c'est-à-dire au niveau de régions possédant une séquence oligopurique sur un brin et une séquence oligopyrimidique sur le brin complémentaire, et y forment localement une triple-hélice. Les bases du troisième brin (l'oligonucléotide) forment des liaisons hydrogène (liaisons Hoogsteen ou Hoogsteen inverse) avec les purines des paires de bases Watson-Crick.

Une utilisation de ce type d'interaction pour isoler un plasmide à été décrite dans l'art antérieur. Ainsi, Ito et al. (PNAS 89 (1992) 495) décrivent l'utilisation d'oligonucléotides biotinylés capables de reconnaître une séquence déterminée d'un plasmide et de former avec celle-ci une triple hélice. Les complexes ainsi formés sont ensuite mis en contact avec des billes magnétiques revêtues de streptavidine. L'interaction entre la biotine et la streptavidine permet alors d'isoler le plasmide par séparation magnétique des billes puis élution. Néanmoins, ce procédé présente certains inconvénients. En particulier, deux interactions spécifiques successives sont nécessaires, la première entre l'oligonucléotide et le plasmide, la deuxième entre le complexe biotinylé et les billes de streptavidine. De plus, la solution finale peut être contaminée par de l'oligonucléotide biotinylé, qui ne peut être utilisé dans une composition pharmaceutique.

Est décrit un procédé amélioré de purification d'ADN faisant appel à ce type d'interaction. Plus particulièrement, le procédé met en oeuvre des oligonucléotides couplés de manière covalente à un support. Ce procédé est particulièrement rapide et il conduit à des rendements et à des degrés de pureté particulièrement élevés. Par ailleurs, il permet de purifier de l'ADN à partir de mélanges complexes comprenant notamment d'autres acides nucléiques, des protéines, des endotoxines (telles que des lipopolysaccharides), des nucléases, etc.... Les supports utilisés peuvent en outre être facilement recyclés, et les ADN obtenus présentent des propriétés de sécurité pharmaceutique améliorées. Enfin, ce procédé implique une seule étape contrairement à l'art antérieur.

Soit un procédé pour la purification d'ADN double-brin, selon lequel on fait passer une solution contenant ledit ADN en mélange avec d'autres composants sur un support sur lequel est couplé de manière covalente un oligonucléotide capable de former par hybridation une triple hélice avec une séquence spécifique présente sur ledit ADN . La séquence spécifique peut être une séquence présente naturellement sur l'ADN double-brin, ou une séquence synthétique introduite artificiellement dans celui-ci.

Les oligonucléotides utilisés dans la présente description sont des oligonucléotides hybridant directement avec l'ADN en double-brin. Ces oligonucléotides peuvent contenir les bases suivantes :
- thymidine (T), qui est capable de former des triplets avec les doublets A.T de l'ADN double-brin (Rajagopal et al, Biochem 28 (1989) 7859);
- adénine (A), qui est capable de former des triplets avec les doublets A.T de l'ADN double-brin;
- guanine (G), qui est capable de former des triplets avec les doublets G.C de l'ADN double-brin;
- cytosine protonée (C+), qui est capable de former des triplets avec les doublets G.C de l'ADN double-brin (Rajagopal et al précitée);
- uracile (U) qui est capable de former des triplets avec les paires de bases A.U ou A.T.

Préférentiellement, l'oligonucléotide utilisé comprend une séquence homopyrimidique riche en cytosines et la séquence spécifique présente sur l'ADN est une séquence homopurique-homopyrimidique. La présence de cytosines permet d'avoir une triple hélice stable à pH acide, où les cytosines sont protonées, et déstabilisée à pH alcalin, où les cytosines sont neutralisées.

Pour permettre la formation d'une triple-hélice par hybridation, il est important que l'oligonucléotide et la séquence spécifique présente sur l'ADN soient complémentaires. A cet égard, pour obtenir les meilleurs rendements et la meilleure sélectivité, on utilise dans le procédé de l'invention un oligonucléotide et une séquence spécifique parfaitement complémentaires. Il peut s'agir en particulier d'un oligonucléotide poly-CTT et d'une séquence spécifique poly-GAA. A titre d'exemple, on peut citer l'oligonucléotide de séquence 5'-GAGGCTTCTTCTTCTTCTTCTTCTT-3' (GAGG(CTT)₇; (SEQ ID n°1), dans lequel les bases GAGG ne forment pas de triple hélice mais permettent d'espacer l'oligonucléotide du bras de couplage; on peut également citer la séquence (CTT)₇ (SEQ ID n°26). Ces oligonucleotides sont capables de former une triple-hélice avec une séquence spécifique comportant des motifs complémentaires (GAA). Il peut s'agir en particulier d'une région comportant 7, 14 ou 17 motifs GAA, comme décrit dans les exemples.

Une autre séquence d'intérêt spécifique est la séquence :
5'-AAGGGAGGGAGGAGAGGAA-3'(SEQ ID n°5).
Cette séquence forme une triple hélice avec les oligonucléotides
5'-AAGGAGAGGAGGGAGGGAA-3'(SEQ ID n°6) ou
5'-TTGGTGTGGTGGGTGGGTT-3'(SEQ ID n°7).

Dans ce cas, l'oligonucléotide se fixe dans une orientation antiparallèle au brin polypurique. Ces triples hélices ne sont stables qu'en présence de Mg2+ (Vasquez et al., Biochemistry, 1995, 34, 7243-7251 ; Beal et Dervan, Science, 1991, 251, 1360-1363).

Comme indiqué ci-avant, la séquence spécifique peut être une séquence présente naturellement sur l'ADN double-brin, ou une séquence synthétique introduite artificiellement dans celui-ci. Il est particulièrement intéressant d'utiliser un oligonucleotide capable de former une triple-hélice avec une séquence présente naturellement sur l'ADN double-brin, par exemple dans l'origine de réplication d'un plasmide ou dans un gène marqueur. A cet égard, la demanderesse a effectué des analyses de séquence de plasmides et a pu montrer que certaines régions de ces ADN, notamment dans l'origine de réplication, pouvaient posséder des régions homopuriquehomopyrimidiques. La synthèse d'oligonucléotides capables de former des triple-hélices avec ces régions homopurique-homopyrimidiques naturelles permet avantageusement d'appliquer le procédé de l'invention a des plasmides non modifiés, notamment des plasmides commerciaux de type pUC, pBR322, pSV, etc. Parmi les séquences homopuriques-homopyrimidiques naturellement présentes sur un ADN double brin, on peut citer une séquence comprenant tout ou partie de la séquence 5'-CTTCCCGAAGGGAGAAAGG-3'(SEQ ID n°2) présente dans l'origine de réplication ColE1 de E. coli. Dans ce cas, l'oligonucléotide formant la triple hélice possède la séquence : 5'-GAAGGGTTCTTCCCTCTTTCC-3'(SEQ ID n°3) et se fixe alternativement sur les deux brins de la double hélice, comme décrit par Beal et Dervan (J. Am. Chem. Soc. 1992, 114, 4976-4982) et Jayasena et Johnston (Nucleic Acids Res. 1992, 20, 5279-5288). On peut aussi citer la séquence 5'-GAAAAAGGAAGAG-3'(SEQ ID n°4) du gène de la β-lactamase du plasmide pBR322 (Duval-Valentin et al., Proc. Natl. Acad. Sci. USA, 1992, 89, 504-508). L'utilisation d'un oligonucléotide capable de former une triple-hélice avec une séquence présente dans une origine de réplication ou un gène marqueur est particulièrement avantageuse car elle permet, avec le même oligonucléotide, de purifier tout ADN contenant ladite origine de réplication ou ledit gène marqueur. Il n'est donc pas nécessaire de modifier le plasmide ou l'ADN double-brin pour lui incorporer une séquence spécifique artificielle.

Bien que des séquences parfaitement complémentaires soient préférées il est entendu toutefois que certains mésappariements peuvent être tolérés entre la séquence de l'oligonucléotide et la séquence présente sur l'ADN, dès lors qu'ils ne conduisent pas à une perte trop grande d'affinité. On peut citer la séquence 5'-AAAAAAGGGAATAAGGG-3'(SEQ ID n°8) présente dans le gène de la β-lactamase de E. coli. Dans ce cas, la thymine interrompant la séquence polypurique peut être reconnue par une guanine du troisième brin, formant ainsi un triplet ATG qui est stable quand il est encadré par deux triplets TAT (Kiessling et al., Biochemistry, 1992, 31, 2829-2834).

Selon un mode de réalisation particulier, les oligonucléotides comprennent la séquence (CCT)n, la séquence (CT)n ou la séquence (CTT)n, dans laquelle n est un nombre entier compris entre 1 et 15 inclus. Il est particulièrement avantageux d'utiliser des séquences de type (CT)n ou (CTT)n. La demanderesse a en effet montré que le rendement de purification était influencé par la quantité de C dans l'oligonucléotide. En particulier, comme indiqué dans l'exemple 7, le rendement de purification augmente lorsque l'oligonucleotide comporte moins de cytosines. Il est entendu que les oligonucléotides de l'invention peuvent également combiner des motifs (CCT), (CT) ou (CTT).

L'oligonucléotide utilisé peut être naturel (composé de bases naturelles, non modifiées) ou modifié chimiquement. En particulier, l'oligonucléotide peut présenter avantageusement certaines modifications chimiques permettant d'augmenter sa résistance ou sa protection vis à vis des nucléases, ou son affinité vis à vis de la séquence spécifique.

On entend aussi par oligonucléotide tout enchainement de nucléosides ayant subi une modification du squelette dans le but de le rendre plus résistant aux nucléases. Parmi les modifications possibles on peut citer, les oligonucléotides phosphorothioates qui sont capable de former des triples hélices avec l'ADN (Xodo et al., Nucleic Acids Res., 1994, 22, 3322-3330), de même que les oligonucléotides possédant des squelettes formacétal ou méthylphosphonate (Matteucci et al., J. Am. Chem. Soc., 1991, 113, 7767-7768). On peut également utiliser les oligonucléotides synthétisés avec des α-anomères de nucléotides, qui forment également des triples hélices avec l'ADN (Le Doan et al., Nucleic Acids Res., 1987, 15, 7749-7760). Une autre modification du squelette est la liaison phosphoramidate. On peut citer par exemple la liaison internucléotidique N3'-P5' phosphoramidate décrite par Gryaznov et Chen, qui donne des oligonucléotides formant avec l'ADN des triples hélices particulièrement stables (J. Am. Chem. Soc., 1994, 116, 3143-3144). Parmi les autres modifications du squelette, on peut citer également l'utilisation de ribonucléotides, de 2'-O-méthylribose, de phosphodiester,...(Sun et Hélène, Curr. Opinion Struct. Biol., 116, 3143-3144). Le squelette phosphoré peut enfin être remplacé par un squelette polyamide comme dans les PNA (Peptide Nucleic Acid), qui peuvent également former des triples hélices (Nielsen et al., Science, 1991, 254, 1497-1500 ; Kim et al., J. Am. Chem. Soc., 1993, 115, 6477-6481)) ou par un squelette à base de guanidine, comme dans les DNG (déoxyribonucleic guanidine, Proc. Natl. Acad. Sci. USA, 1995, 92, 6097-6101), analogues polycationiques de l'ADN, qui forment également des triples hélices.

La thymine du troisième brin peut aussi être remplacée par une 5-bromouracile, ce qui augmente l'affinité de l'oligonucléotide pour l'ADN (Povsic et Dervan, J. Am. Chem. Soc., 1989, 111, 3059-3061). Le troisième brin peut également contenir des bases non naturelles, parmi lesquelles on peut citer la 7-déaza-2'-déoxyxanthosine (Milligan et al., Nucleic Acids Res., 1993, 21, 327-333), la 1-(2-déoxy-β-D-ribofuranosyl)-3-méthyl-5-amino-1*H*-pyrazolo[4,3-*d*]pyrimidine-7-one (Koh et Dervan, J. Am. Chem. Soc., 1992, 114, 1470-1478), la 8-oxoadénine, la 2-aminopurine, la 2'-O-méthyl-pseudoisocytidine, ou toute autre modification connue de l'homme du métier (voir pour revue Sun et Hélène, Curr. Opinion Struct. Biol., 1993, 3, 345-356).

Un autre type de modification de l'oligonucléotide a plus particulièrement pour objet d'améliorer l'interaction et/ou l'affinité entre l'oligonucléotide et la séquence spécifique. En particulier, une modification tout à fait avantageuse consiste à méthyler les cytosines de l'oligonucléotide (cf. exemple 5). L'oligonucléotide ainsi méthylé présente la propriété remarquable de former une triple hélice stable avec la séquence spécifique dans des zones de pH plus proches de la neutralité(≥ 5). Il permet donc de travailler à des pH plus élevés que les oligonucléotides de l'art antérieur, c'est à dire à des pH où les risques de dégradation de l'ADN plasmidique sont bien inférieurs.

La longueur de l'oligonucléotide utilisé dans le procédé décrit est d'au moins 3 bases, et de préférence, comprise entre 5 et 30. On utilise de manière avantageuse un oligonucléotide de longueur supérieure à 10 bases. La longueur peut être adaptée au cas par cas par l'homme du métier en fonction de la sélectivité et de la stabilité de l'interaction recherchées.

Les oligonucléotides décrits peuvent être synthétisés par toute technique connue. En particulier, ils peuvent être préparés au moyen de synthétiseurs d'acides nucléiques. Toute autre méthode connue de l'homme du métier peut bien évidemment être utilisée.

Pour permettre son couplage covalent sur le support, l'oligonucléotide est généralement fonctionnalisé. Ainsi, il peut être modifié par un groupement terminal thiol, amine ou carboxyle, en position 5' ou 3'. En particulier, l'ajout d'un groupe thiol, amine ou carboxyle permet, par exemple, de coupler l'oligonucléotide sur un support portant des fonctions disulfure, maléimide, amine, carboxyle, ester, époxyde, bromure de cyanogène ou aldéhyde. Ces couplages se forment par établissement de liaisons disulfure, thioether, ester, amide ou amine entre l'oligonucléotide et le support. Toute autre méthode connue de l'homme du métier peut être utilisée, telle que des réactifs de couplage bifonctionnels, par exemple.

Par ailleurs, pour améliorer l'hybridation avec l'oligonucléotide couplé, il peut être avantageux que l'oligonucléotide contienne un "bras" et une séquence de bases "espaceur". L'utilisation d'un bras permet en effet de fixer l'oligonucléotide à une distance choisie du support permettant d'améliorer ses conditions d'interaction avec l'ADN. Le bras est avantageusement constitué d'une chaîne carbonée linéaire, comprenant 1 à 18, et de préférence 6 ou 12 groupes (CH2), et d'une amine qui permet la liaison à la colonne. Le bras est relié a un phosphate de l'oligonucléotide ou d'un "espaceur" composé de bases n'interférant pas avec l'hybridation. Ainsi, "l'espaceur" peut comprendre des bases puriques. A titre d'exemple, "l'espaceur" peut comprendre la séquence GAGG. Le bras est avantageusement composé d'une chaîne carbonée linéaire comprenant 6 ou 12 atomes de carbones.

Différents types de supports peuvent être utilisés. Il peut s'agir de supports de chromatographie fonctionnalisés, en vrac ou préconditionnés en colonne, de surfaces plastiques fonctionnalisées ou de billes de latex fonctionnalisées, magnétiques ou non. Il s'agit préférentiellement de supports de chromatographie. A titre d'exemple, les supports de chromatographie pouvant être utilisés sont l'agarose, l'acrylamide ou le Dextran ainsi que leurs dérivés (tels que Séphadex, Sépharose, Superose,...), les polymères tels que le poly(styrènedivinylbenzène), ou la silice greffée ou non greffée, par exemple. Les colonnes de chromatographie peuvent fonctionner en mode de diffusion ou de perfusion.

Pour obtenir de meilleurs rendement de purification, il est particuliérement avantageux d'utiliser, sur le plasmide, une séquence comportant plusieurs positions d'hybridation avec l'oligonucléotide. La présence de plusieurs positions d'hybridation favorise en effet les interactions entre ladite séquence et l'oligonucléotide, ce qui conduit à améliorer les rendements de purification. Ainsi pour un oligonucléotide comportant n répétitions de motifs (CCT), (CT) ou (CTT), il est préférable d'utiliser une séquence d'ADN comportant au moins n motifs complémentaires et , de préférence, n+1 motifs complémentaires. Une séquence portant n+1 motifs complémentaires offre ainsi deux positions d'hybridation à l'oligonucléotide. Avantageusement, la séquence d'ADN comporte jusqu'a 11 positions d'hybridation, c'est à dire n+10 motifs complémentaires.

Le procédé selon la présente description peut être utilisé pour purifier tout type d'ADN double brin. Il s'agit par exemple d'ADN circulaire, tel qu'un plasmide portant généralement un ou plusieurs gènes d'intérêt thérapeutique. Ce plasmide peut porter également une origine de réplication, un gène marqueur, etc.... Le procédé peut être appliqué directement à un lysat cellulaire. Dans ce mode de réalisation, le plasmide, amplifié par transformation puis culture cellulaire, est purifié directement après lyse des cellules. Le procédé peut également être appliqué à un lysat clair, c'est à dire au surnageant obtenu après neutralisation et centrifugation du lysat cellulaire. Il peut bien évidement être appliqué également à une solution prépurifiée par des méthodes connues. Ce procédé permet aussi de purifier de l'ADN, linéaire ou circulaire, portant une séquence d'intérêt, à partir d'un mélange comprenant des ADN de différentes séquences. Le procédé selon l'invention peut également être utilisé pour la purification d'ARN double brin.

Le lysat cellulaire peut être un lysat de cellules procaryotes ou eucaryotes. S'agissant de cellules procaryotes, on peut citer par exemple les bactéries E. coli, B. subtilis, S. typhimurium ou Streptomyces. S'agissant de cellules eucaryotes, on peut citer les cellules animales, les levures, les champignons, etc..., et plus particulièrement, les levures Kluyveromyces ou Saccharomyces ou les cellules COS, CHO, C127, NIH3T3, etc....

Le procédé décrit est particulièrement avantageux puisqu'il permet d'obtenir, de manière rapide et simple, de l'ADN plasmidique de très haute pureté. En particulier, comme illustré dans les exemples, ce procédé permet de séparer efficacement l'ADN plasmidique considéré de composants contaminants, tels que l'ADN chromosomique fragmenté, les endotoxines, les protéines, les nucléases, etc.... Plus particulièrement, le procédé décrit permet d'obtenir des préparation d'ADN double brin, notamment plasmidique, ayant une teneur en ADN chromosomique inférieure ou égale à 0.5%. Encore plus préférentiellement, les préparations d'ADN obtenues ont une teneur en ADN chromosomique inférieure ou égale à 0.2%. Sont décrites donc des compositions comprenant de l'ADN plasmidique, utilisables pharmaceutiquement notamment en thérapie génique ou cellulaire. A cet égard, est également décrite une composition pharmaceutique comprenant de l'ADN double brin, linéaire ou plasmidique, préparé selon le procédé décrit ci-avant.

Egalement décrites des préparations d'ADN plasmidiques ayant une teneur en ADN chromosomique inférieure ou égale à 0.5%, préférentiellement à 0.2% encore plus préférentiellement à 0.1%.

Les compositions peuvent comporter l'ADN plasmidique "nu" ou associé à des vecteurs de transport tels que les liposomes, les nanoparticules, les lipides cationiques, les polymères, des protéines ou virus recombinants, etc.

La présente demande sera décrite plus en détail à l'aide des exemples qui suivent, qui doivent être considérés comme illustratifs.et non limitatifs.

### Techniques générales de clonage et de biologie moléculaire.

Les méthodes classiques de biologie moléculaire telles que les digestions par des enzymes de restriction, l'électrophorèse sur gel, la transformation dans E. coli, la précipitation des acides nucléiques etc., sont décrites dans la littérature (Maniatis et al., T., E. F. Fritsch, and J. Sambrook. 1989. Molecular cloning: a laboratory manual, second edition. Cold Spring Harbor Laboratory, Cold Spring Harbor Laboratory Press, New York ; Ausubel F. M., R. Brent, R. E. Kinston, D. D. Moore, J. A. Smith, J. G. Seidman and K. Struhl. 1987. Current protocols in molecular biology 1987-1988. John Willey and Sons, New York. ). Les séquences nucléotidiques ont été déterminées par la méthode de terminaison de chaînes en suivant le protocole déjà présenté (Ausubel et al., 1987).

Les enzymes de restriction ont été fournies par New-England Biolabs, Beverly, MA (Biolabs).

Pour les ligatures, les fragments d'ADN sont incubés dans un tampon Tris-HCl pH 7.4 50 mM, MgCl₂ 10 mM, DTT 10 mM, ATP 2 mM, en présence d'ADN ligase du phage T4 (Biolabs, ).

Les oligonucléotides sont synthétisés en utilisant la chimie des phosphoramidites protégés en β par un groupement cyanoéthyl (Sinha, N. D., J. Biernat, J. McManus and H. Köster. 1984. Polymer support oligonucleotide synthesis, XVIII: Use of β-cyanoethyl-N,N-dialkylamino-/N-morpholino phosphoramidite of déoxynucleosides for the synthesis of DNA fragments simplifying deprotection and isolation of the final product. Nucl. Acids Res., 12, 4539-4557 ; Giles, J. W. 1985. Advances in automated DNA synthesis. Am. Biotechnol., Nov./Dec.) avec le synthétiseur automatique d'ADN Biosearch 8600 en utilisant les recommandations du fabricant.

Les ADN ligaturés ou à tester pour leur efficacité de transformation sont utilisés pour transformer la souche rendue compétente: E. coli DH5α [F'/ endA1, hsdR17, supE44, thi-1, recA1, gyrA96, relA1, Δ(lacZYA-argF)U169, deoR, Φ80dlac(lacZΔM15)]

Les minipréparations d'ADN plasmidique sont faites suivant le protocole de Klein et al., 1980.

Le milieu de culture LB est utilisé pour la croissance des souches d'E. coli (Maniatis et al., 1982). Les souches sont incubées à 37°C. Les bactéries sont étalées sur des boites de milieu LB supplémentées avec des antibiotiques appropriés.

### Exemple 1.

### 1.1. Préparation de la colonne

### Matériel :

La colonne utilisée est une colonne HiTrap activée au NHS (N-hydroxysuccinimide, Pharmacia) de 1 ml, connectée sur une pompe péristaltique (débit < 1 ml/min).

L'oligonucléotide spécifique utilisé possède un groupement NH₂ en 5'. Sa séquence est la suivante :
5'-GAGGCTTCTTCTTCTTCTTCTTCTT-3'(SEQ ID n°1)

Les tampons utilisés dans cet exemple sont les suivants :
Tampon de couplage : NaHCO₃ 0,2 M, NaCl 0,5 M, pH 8,3.
Tampon A : éthanolamine 0,5 M, NaCl 0,5 M, pH 8,3.
Tampon B : acétate 0,1 M, NaCl 0,5 M, pH 4.

### Méthode:

La colonne est lavée par 6 ml de HCl 1 mM, puis l'oligonucléotide dilué dans le tampon de couplage (50 nmoles dans 1 ml) est appliqué sur la colonne et laissé 30 minutes à température ambiante. La colonne est lavée 3 fois successives par 6 ml de tampon A puis 6 ml de tampon B. L'oligonucléotide est ainsi lié covalemment à la colonne par une liaison CONH. La colonne est stockée à 4°C dans du PBS 0,1% NaN₃ et peut être utilisée au moins quatre fois.

### 1.2. Construction des plasmides

Les deux oligonucléotides suivants ont été synthétisés.
oligonucléotide 4817:
5'-GATCCGAAGAAGAAGAAGAAGAAGAAGAAGAAGAAGAAGAAGAAGAAGA AGAAGAAGG-3'(SEQ ID n°9)
oligonucléotide 4818:
5'-AATTCCTTCTTCTTCTTCTTCTTCTTCTTCTTCTTCTTCTTCTTCTTCTTCTTCT TCG-3'(SEQ ID n°10)

Ces oligonucléotides, une fois hybridés et clones sur un plasmide, apportent sur le plasmide correspondant une séquence homopurique-homopyrimidique (GAA)₁₇, comme décrit ci-dessus.

La séquence correspondant à ces deux oligonucléotides hybridés a été clonée au multisite de clonage du plasmide pBKS+ (Stratagene Cloning System, La Jolla CA), qui porte un gène de résistance à l'ampicilline. Pour ce faire, les oligonucléotides ont été hybridés de la manière suivante. Un µg de ces deux oligonucléotides ont été mis ensemble dans 40 ml d'un tampon final 50 mM Tris-HCl pH 7.4, 10 mM MgCl₂. Ce mélange a été chauffé à 95°C, puis il a été placé à la température ambiante de manière que la température s'abaisse lentement. Dix ng du mélange d'oligonucléotides hybridés ont été ligaturés avec 200 ng du plasmide pBKS+ (Stratagene Cloning System, La Jolla CA) digérés par BamHI et EcoRI dans 30 µl final. Après ligature une aliquote a été transformée dans DH5α. Les mélanges de 'transformation ont été étalés sur milieu L supplémenté avec de l'ampicilline (50 mg/l) et du X-gal (20 mg/l). Les clones recombinants doivent présenter une absence de coloration bleu sur ce milieu contrairement au plasmide parent (pBKS+) qui permet l'α-complémentation du fragment ω de la β-galactosidase d'E. coli. Après minipréparation d'ADN plasmidique de 6 clones, ils ont tous présentés la disparition du site PstI, situé entre les sites EcoRI et BamHI de pBKS+, et une augmentation de poids moléculaire de la bande PvuII de 448 pb contenant le multisite de clonage. Un clone a été retenu et le plasmide correspondant a été nommé pXL2563. La séquence clonée a été vérifiée par séquence en utilisant le primer -20 (5'-TGACCGGCAGCAAAATG-3'(SEQ ID n°11)) (Viera J. and J. Messing. 1982. The pUC plasmids, an M13mp7-derived system for insertion mutagenesis and sequencing with synthetic universal primers. Gene, 19, 259-268). du plasmide pBKS+ (Stratagene Cloning System, La Jolla CA).

Le plasmide pXL2563 a été purifié selon le kit Wizard Megaprep (Promega Corp., Madison, WI) en suivant les recommandations du fournisseur. Cette préparation d'ADN plasmidique a été utilisée par la suite dans des exemples décrits ci-après.

### 1.3. Purification de plasmide.

### Matériel :

Le plasmide pXL2563 (décrit en 1.2) a été purifié sur la colonne HiTrap couplée à l'oligonucléotide décrite en 1.1., à partir d'une solution contenant également le plasmide pBKS+. Les tampons utilisés lors de cette purification sont les suivants :
Tampon F : NaCl 2M, acétate 0,2 M, pH 4,5 à 5.
Tampon E :Tris 1 M, HCl pH 9, EDTA 0,5 mM.

### Méthode :

La colonne est lavée par 6 ml de tampon F, puis les plasmides (20 µg de pXL2563 et 20 µg de pBKS+ dans 400 µl de tampon F) sont appliqués sur la colonne et incubés deux heures à température ambiante. La colonne est lavée par 10 ml de tampon F puis l'élution se fait par du tampon E. Les plasmides sont détectés après électrophorèse sur gel d'agarose 1% et coloration au bromure d'éthidium. La proportion des plasmides dans les solutions est estimée par mesure de leur activité transformante sur E.coli.

### Résultat :

En partant d'un mélange contenant 30% de pXL2563 et 70% de pBKS+, on récupère en sortie de colonne une solution à 100% de pXL2563. La pureté, estimée par le rapport de D.O. à 260 et 280 nm, passe de 1,9 à 2,5, ce qui indique que l'on élimine des protéines contaminantes par cette méthode.

### Exemple 2.

2.1 - Cet exemple décrit une expérience de purification d'ADN plasmidique. Le couplage de l'oligonucléotide (5'-GAGGCTTCTTCTTCTTCTTCTTCTT-3'(SEQ ID n°1)) sur la colonne est effectué comme indiqué dans l'exemple 1.Pour le couplage, l'oligonucléotide est modifié à l'extrémité 5' par un groupement amine lié au phosphate de l'espaceur par un bras contenant 6 atomes de carbone (Oligonucléotide modifié Eurogentec SA Belgique). Le plasmide pXL2563 a été purifié à l'aide du kit Wizard Megaprep (Promega Corp., Madison, WI) en suivant les recommandations du fournisseur. Les tampons utilisés dans cet exemple sont les suivants:
Tampon F : NaCl 0-2M, acétate 0,2 M, pH 4,5-5.
Tampon E :Tris 1 M, HCl pH 9, EDTA 0,5 mM.

La colonne est lavée par 6 ml de tampon F, puis 100 µg de plasmide pXL2563 dilués dans 400 µl de tampon F sont appliqués sur la colonne et incubés deux heures à température ambiante. La colonne est lavée par 10 ml de tampon F puis l'élution se fait avec du tampon E. Le plasmide est quantifié par mesure de densité optique à 260 nm. Dans cet exemple, la fixation est réalisée dans un tampon dont la molarité en NaCl varie de 0 à 2M (tampon F). Le rendement de purification diminue lorsque la molarité en NaCl décroît. Le pH du tampon de fixation peut varier de 4,5 à 5, le rendement de purification étant meilleur à 4,5. On peut également utiliser un autre tampon d'élution de pH basique : ainsi l'élution a été réalisée avec un tampon borate 50 mM, pH 9, EDTA 0,5 mM.

2.2 - On procède au couplage de l'oligonucléotide : (5'-GAGGCTTCTTCTTCTTCTTCTTCTT-3'(SEQ ID n°1) sur la colonne comme indiqué dans l'exemple 1. Le plasmide pXL2563 a été purifié à l'aide du kit Wizard Megaprep (Promega Corp., Madison, WI) en suivant les recommandations du fournisseur. Les tampons utilisés dans cet exemple sont les suivants:
Tampon F : NaCl 0,1M, acétate 0,2 M, pH 5.
Tampon E :Tris 1 M, HCl pH 9, EDTA 0,5 mM.

La colonne est lavée par 6 ml de tampon F, puis 100 µg de plasmide pXL2563 dilués dans 400 µl de tampon F sont appliqués sur la colonne et incubés une heure à température ambiante. La colonne est lavée par 10 ml de tampon F puis l'élution se fait avec du tampon E. On mesure le taux d'ADN génomique ou chromosomique d'E. coli présent dans les échantillons de plasmide avant et après passage sur la colonne d'oligonucléotide. Cet ADN génomique est quantifié par PCR en utilisant des amorces dans le gène galK d'E. coli. Selon le protocole suivant: La séquence de ces amorces est décrite par Debouck et al. (Nucleic Acids Res., 1985, 13, 1841-1853) :
5'-CCG AAT TCT GGG GAC CAA AGC AGT TTC-3'(SEQ ID N° 24)
et 5'-CCA AGC TTC ACT GTT CAC GAC GGG TGT-3'(SEQ ID N°25).

Le milieu réactionnel comprend, dans 25 µl de tampon PCR (Promega France, Charbonnières) : 1,5 mM MgCl₂ ; 0,2 mM dXTP (Pharmacia, Orsay) ; 0,5 µM en amorce ; 20 U/ml Taq polymérase (Promega). La réaction est effectuée suivant la séquence :
- 5 min. à 95°C
- 30 cycles de
   10 sec. à 95°C
   30 sec. à 60°C
   1 min. à 78°C
- 10 min. à 78°C.

Le fragment d'ADN amplifié, d'une longueur de 124 paires de bases, est séparé par électrophorèse sur gel d'agarose à 3% en présence de SybrGreen I (Molecular Probes, Eugene , USA), puis quantifié par référence à une gamme d'ADN génomique Ultrapur d'E. coli, souche B (Sigma, réf. D4889).

On a 1% d'ADN chromosomique dans l'échantillon déposé sur la colonne, et 0,2% dans l'échantillon purifié sur la colonne d'oligonucléotide.

### Exemple 3. Expérience sur lysat clair

Cet exemple décrit la purification d'ADN plasmidique à partir d'un lysat clair de culture bactérienne, à l'échelle dite "miniprep": 1,5 ml d'une culture de nuit, de souches DH5α contenant le plasmide pXL2563, sont centrifugés et le culot est remis en suspension dans 100 µl de glucose 50 mM, Tris 25 mM HCl pH 8, EDTA 10 mM. On ajoute 200 µl de NaOH 0,2 M, SDS 1%, les tubes sont agités par inversion, puis on ajoute 150 µl d'acétate de potassium 3 M, pH 5 et les tubes sont agités par inversion. Après centrifugation, le surnageant est récupéré et chargé sur la colonne d'oligonucléotide obtenue comme décrit à l'exemple 1. La fixation, les lavages et l'élution sont identiques à ceux décrits dans l'exemple 1. On récupère environ 1 µg de plasmide à partir de 1,5 ml de culture. Le plasmide obtenu, analysé par électrophorèse sur gel d'agarose et coloration au bromure d'éthidium, se présente sous la forme d'une seule bande d'ADN circulaire "super enroulé". Aucune trace d'ADN de haut poids moléculaire (chromosomique), ni d'ARN n'est détectable dans le plasmide purifié par cette méthode. Le rapport des densités optiques à 260 et 280 nm est supérieur à 2.

### Exemple 4.

4.1 : Cet exemple décrit une expérience de purification d'ADN plasmidique réalisée dans les mêmes conditions que l'exemple 3, à partir de 20 ml de culture bactérienne de souches DH5α contenant le plasmide pXL2563. Le culot cellulaire est repris dans 1,5 ml de glucose 50 mM, Tris 25 mM HCl pH 8, EDTA 10 mM. La lyse est faite par 2 ml de NaOH 0,2 M, SDS 1%, et la neutralisation par 1,5 ml d'acétate de potassium 3 M, pH 5. L'ADN est ensuite précipité par 3 ml de propranolol-2, le culot est repris dans 0,5 ml d'acétate de sodium 0,2 M pH 5, NaCl 0,1M et chargé sur la colonne d'oligonucléotide obtenue comme décrit à l'exemple 1. La fixation, le lavage de la colonne et l'élution se font comme décrit à l'exemple 1, à l'exception du tampon de lavage dont la molarité en NaCl est 0,1M. On obtient environ 16 µg d'ADN plasmidique. Le plasmide obtenu, analysé par électrophorèse sur gel d'agarose et coloration au bromure d'éthidium, se présente sous la forme d'une seule bande d'ADN circulaire "super enroulé". Aucune trace d'ADN de haut poids moléculaire (chromosomique), ni d'ARN n'est détectable dans le plasmide purifié. La digestion du plasmide par une enzyme de restriction donne une seule bande au poids moléculaire attendu de 3 kilobases. La concentration en protéines dans les échantillons passe de 125 µg/ml dans le lysat clair à moins de 1 µg/ml dans le plasmide purifié (dosage Micro-BCA, Pierce). La concentration en endotoxines, estimée par le dosage LAL (Biosepra) est divisée par un facteur supérieur à 10 dans le plasmide purifié, par rapport au lysat clair de départ.

4.2 : Le plasmide utilisé comporte une cassette contenant le promoteur du Cytomégalovirus, le gène codant pour la luciférase, et la séquence homopurique-homopyrimidique (GAA)₁₇ issue du plasmide pXL2563. La souche DH1 (Maniatis et al., 1989) contenant ce plasmide est cultivée en fermenteur de 7 litres. Un lysat clair est préparé à partir de 200 grammes de cellules : le culot cellulaire est repris dans 2 litres de Tris 25 mM, pH 6,8, glucose 50 mM, EDTA 10 mM, auxquels on ajoute 2 litres de NaOH 0,2 M, SDS 1%. Le lysat est neutralisé par addition d'un litre d'acétate de potassium 3M.Après diafiltration 4 ml de ce lysat sont déposés sur une colonne HiTrap-NHS de 5 ml, couplée à l'oligonucléotide de séquence 5'-GAGGCTTCTTCTTCTTCTTCTTCTT-3' (SEQ ID n° 1), selon la méthode décrite à l'exemple 1.1. Le lavage et l'élution se font comme décrit à l'exemple 1.On récupère environ 400 microgrammes de plasmide. Le taux d'ADN génomique dans cet échantillon, mesuré par la technique décrite à l'exemple 2.2, est de 0,1%.

### Exemple 5. Utilisation d'un oligonucléotide modifié

Cet exemple décrit l'utilisation d'un oligonucléotide portant des cytosines méthylées.
La séquence de l'oligonucléotide utilisée est la suivante :
5'-GAGG^{Me}CTT^{Me}CTT^{Me}CTT^{Me}CTT^{Me}CTT^{Me}CTT^{Me}CTT-3'(SEQ ID n° 12)

Cet oligonucléotide possède un groupement NH₂ en 5'. ^{Me}C = 5 méthylcytosine. Cet oligonucléotide permet de purifier le plasmide pXL2563 dans les conditions de l'exemple 1, avec un tampon de fixation de pH 5 (on diminue ainsi le risque de dégradation du plasmide).

### Exemple 6 :

Dans les exemples précédents, l'oligonucléotide utilisé est modifié à l'extrémité 5'-terminale avec un groupement amine lié au phosphate par un bras contenant 6 atomes de carbone: NH₂-(CH₂)₆. Dans cet exemple, le groupement amine est lié au phosphate de l'extrémité 5'-terminale par un bras à 12 atomes de carbone: NH₂-(CH₂)₁₂. Le couplage de l'oligonucléotide et le passage sur la colonne sont faits comme indiqué à l'exemple 2 avec un tampon F : NaCl 2M, acétate 0,2 M, pH 4,5. Cet oligonucléotide permet d'avoir de meilleurs rendements de purification : on obtient un rendement de 53%, alors qu'avec l'oligonucléotide à 6 atomes de carbone, dans les mêmes conditions ce rendement est de l'ordre de 45%.

### Exemple 7 :

En suivant la stratégie de clonage décrite à l'exemple 1.2, deux autres plasmides portant des séquences homopuriques-homopyrimidiques ont été construits : le plasmide pXL2725 qui contient la séquence (GGA)₁₅ et le plasmide pXL2726 qui contient la séquence (GA)₂₅.

### Exemple 7.1 Construction des plasmides

Les plasmides pXL2725 et pXL2726, analogues au plasmide pXL2563, ont été construits selon la stratégie de clonage décrite à l'exemple 1.2, en utilisant les couples d'oligonucléotides suivants:
5986 : 5'-GATCC(GA)₂₅GGG-3'(SEQ ID n°13)
5987 : 5'-AATTCCC(TC)₂₅G-3'(SEQ ID n°14)
5981 : 5'-GATCC(GGA)₁₇GG-3'(SEQ ID n°15)
5982 : 5'-AATT(CCT)₁₇CCG-3'(SEQ ID n°16)

Le couple d'oligonucléotides 5986 et 5987 a été utilisé pour construire le plasmide pXL2726 en clonant les oligonucléotides aux sites BamHI et EcoRI de pBKS+ (Stratagene Cloning System, La Jolla CA) tandis que les oligonucléotides 5981 et 5982 ont été utilisés pour la constuction du plasmide pXL2725. Les mêmes conditions expérimentales que pour la construction du plasmide pXL2563 ont été utilisées et seuls les couples d'oligonucléotides ont été changés. De même, les séquences clonées ont été vérifiées par séquençage sur les plasmides. Ceci a permis de voir que le plasmide pXL2725 possède une modification par rapport à la séquence attendue, au lieu de la séquence GGA répétée 17 fois, il y a GGAGA(GGA)₁₅(SEQ ID n°17).

### Exemple 7.2 : Préparation des colonnes et Purification

Les oligonucléotides formant des triples hélices avec ces séquences homopuriques ont été couplés à des colonnes HiTrap selon la technique décrite à l'exemple 1.1. Il s'agit de l'oligonucléotide de séquence
5'-AATGCCTCCTCCTCCTCCTCCTCCT-3'(SEQ ID n°18) pour la purification du plasmide pXL2725, et de l'oligonucléotide de séquence
5'-AGTGCTCTCTCTCTCTCTCTCTCTCT-3'(SEQ ID n° 19) pour la purification du plasmide pXL2726.

Les deux colonnes ainsi obtenues ont permis de purifier les plasmides correspondants selon la technique décrite à l'exemple 2, avec les tampons suivants :
Tampon F : NaCl 2M, acétate 0,2 M, pH 4,5.
Tampon E :Tris 1 M, HCl pH 9, EDTA 0,5 mM.
Les rendements obtenus sont de 23% et 31% pour pXL2725 et pXL2726, respectivement.

### Exemple 8 :

Cet exemple illustre l'influence de la longueur de la séquence spécifique, présente sur le plasmide, sur les rendements de purification.

### Exemple 8.1 : Construction des plasmides

Le gène reporter utilisé dans ces expériences pour mettre en évidence l'activité des compositions de l'invention est le gène codant pour la luciférase (Luc).

Le plasmide pXL2621 comporte une cassette contenant le promoteur du Cytomégalovirus (CMV) de 661 bp extraite de pcDNA3 (Invitrogen, Corp., San Diego, CA) par coupure avec les enzymes de restriction MluI et HindIII, clonée en amont du gène codant pour la luciférase, aux sites MluI et HindIII, dans le vecteur pGL basic Vector (Promega Corp., Madisson, WI). Ce plasmide a été construit en utilisant les techniques standard de biologie moléculaire.

Les plasmides pXL2727-1 et pXL2727-2 ont été construits de la manière suivante.

Deux microgrammes du plasmide pXL2621 ont été linéarisés par BamHI; l'enzyme a été inactivée par un traitement de 10 mn à 65°C; parallèlement les oligonucléotides 6006 et 6008 ont été hybridés comme cela est décrit pour la construction du plasmide pXL2563.
6006 5-GATCT(GAA)₁₇CTGCAGATCT-3'(SEQ-ID n°20)
6008 5'-GATCAGATCTGCAG(TTC)₁₇A-3'(SEQ ID n°21)
Ce mélange d'hybridation a été cloné aux extrémités BamHI du plasmide pXL2621 et après transformation dans DH5α des clones recombinants ont été repérés par analyse par restriction enzymatique par PstI puisque les oligonucléotides introduisent un site PstI. Deux clones ont été retenus et la séquence nucléotidique du fragment cloné a été vérifiée en utilisant le primer (6282, 5'-ACAGTCATAAGTGCGGCGACG-3'(SEQ ID n°22)) comme amorce de réaction de séquence (Viera J. and J. Messing, 1982. The pUC plasmids an M13mp7-derived system for insertion mutagenesis and sequencing with synthetic universal primers. Gene 19:259-268)

Le premier clone (pXL2727-1) contient la séquence GAA répétée 10 fois. Le deuxième (pXL2727-2) contient la séquence 5'-GAAGAAGAG(GAA)₇GGAAGAGAA-3'(SEQ ID n°23).

### Exemple 8.2 : Préparation des colonnes et Purification

On utilise une colonne telle que celle décrite à l'exemple 1 et qui est couplée à l'oligonucléotide 5'-GAGGCTTCTTCTTCTTCTTCTTCTT-3`(SEQ ID n°1).

Le plasmide pXL2727-1 porte 14 répétitions de la séquence GAA. L'oligonucléotide décrit ci-dessus, qui ne compte que 7 répétitions de la séquence d'hybridation correspondante CTT, peut donc s'hybrider avec le plasmide en 8 positions différentes. Le plasmide pXL2727-2 par contre, possède une séquence hybridante (GAA)₇ de la même longueur que celle de l'oligonucléotide fixé sur la colonne. Cet oligonucléotide ne peut donc s'hybrider qu'en une seule position sur le pXL2727-2.

L'expérience est identique à celle décrite dans l'exemple 2 avec les tampons suivants :
Tampon F : NaCl 2M, acétate 0,2 M, pH 4,5.
Tampon E :Tris 1 M, HCl pH 9, EDTA 0,5 mM.
Le rendement de purification est de 29% avec le plasmide pXL2727-1 et de 19% avec le pXL2727-2

### Exemple 8.3 : transfection in vitro de celluless de mammifères.

Les cellules utilisées sont des cellules NIH 3T3, ensemencées la veille de l'expérience dans des plaques de culture à 24 puits, à raison de 50.000 cellules/puits. Le plasmide est dilué dans du NaCl 150 mM et mélangé avec le lipofectant RPR115335. On utilise un rapport de charges positives du lipofectant/charges négatives de l'ADN égal à 6. Le mélange est vortexé, laissé dix minutes à température ambiante, dilué dans du milieu dépourvu de sérum de veau foetal, puis ajouté aux cellules, à raison de 1µg d'ADN par puits de culture. Après deux heures à 37°C, on ajoute 10% volume à volume de sérum de veau foetal et les cellules sont incubées 48 heures à 37°C en présence de 5% de CO2. Les cellules sont lavées deux fois au PBS et l'activité luciférase est mesurée selon le protocole décrit (kit Promega, Promega Corp. Madison, WI), sur un luminomètre Lumat LB9501 (EG et G Berthold, Evry). Le plasmide pXL2727-1, purifié tel que décrit à l'exemple 8.2, donne des rendements de transfection deux fois plus importants que ceux obtenus avec le même plasmide purifié à l'aide du kit Wizard Megaprep (Promega Corp. Madison, WI)

## Revendications

1. Support pour la purification d'un ADN double brin, **caractérisé en ce que** ledit support est couplé de manière covalente à un oligonucléotide capable de former par hybridation une triple hélice avec une séquence spécifique de l'ADN double brin, ledit oligonucléotide comprenant une séquence homopyrimidique choisie parmi la séquence (CCT)ₙ, la séquence (CT)ₙ, et la séquence (CTT)ₙ, dans laquelle n est un nombre entier compris entre 1 et 15, **caractérisé en ce que** l'oligonucléotide contient un bras constitué d'une chaine carbonée linéaire (CH₂)ₙ, où n est un nombre entier compris entre 1 et 18, et d'une amine capable de former une liaison covalente avec le support, et un espaceur composé de bases n'interférant pas avec l'hybridation, ledit espaceur étant relié à la séquence homopyrimidique et au bras, et ledit bras étant lié au support.

2. Support selon la revendication 1, **caractérisé en ce que** l'oligonucléotide comprend des cytosines méthylées.

3. Support selon l'une quelconque des revendications précédentes, **caractérisé en ce que** le support est un support de chromatographie fonctionnalisé, un support constitué de surfaces plastiques fonctionnalisées ou un support constitué de billes de latex fonctionnalisées.

4. Support selon la revendication 3, **caractérisé en ce que** le support est un support de chromatographie fonctionnalisé choisi parmi l'agarose, l'acrylamide, le Dextran, les dérivés de l'acrylamide, les dérivés du Dextran, le poly(styrènedivinylbenzène), la silice greffée et la silice non greffée.

5. Support selon l'une quelconque des revendications précédentes, **caractérisé en ce que** l'oligonucléotide comprend une séquence choisie parmi les séquences SEQ ID NO: 1, SEQ ID NO: 3, SEQ ID NO: 12, SEQ ID NO: 18, SEQ ID NO: 19, et SEQ ID NO: 26.

6. Procédé de préparation d'un support selon la revendication 1, consistant à coupler de manière covalente l'oligonucléotide sur ledit support.

7. Procédé selon la revendication 6, **caractérisé en ce que** l'oligonucléotide comprend des cytosines méthylées.

8. Procédé selon l'une quelconque des revendications 6 ou 7, **caractérisé en ce que** l'oligonucléotide est fonctionnalisé.

9. Procédé selon la revendication 7, **caractérisé en ce que** l'oligonucléotide est modifié par un groupement terminal thiol, amine, ou carboxyle en position 5' ou en 3'.

10. Procédé selon l'une quelconque des revendications 6 à 9, **caractérisé en ce que** le support est un support de chromatographie fonctionnalisé, un support constitué de surfaces plastiques fonctionnalisées ou de billes de latex fonctionnalisées.

11. Procédé selon la revendication 10, **caractérisé en ce que** le support est un support de chromatographie fonctionnalisé choisi parmi l'agarose, l'acrylamide, le Dextran, les dérivés de l'acrylamide, les dérivés du Dextran, le poly(styrènedivinylbenzène), la silice greffée et la silice non greffée.

12. Procédé selon l'une des revendications 10 ou 11, **caractérisé en ce que** le support porte des fonctions disulfure, maléimide, amine, carboxyle, ester, époxyde, bromure de cyanogène ou aldéhyde.

13. Procédé de préparation selon l'une quelconque des revendications 6 à 12, **caractérisé en ce que** ledit oligonucléotide comprend une séquence choisie parmi les séquences SEQ ID NO : 1, SEQ ID NO : 3, SEQ ID NO: 12, SEQ ID NO: 18, SEQ ID NO: 19, et SEQ ID NO: 26.

## Patentansprüche

1. Träger für die Reinigung einer doppelsträngigen DNA, **dadurch gekennzeichnet, dass** der Träger kovalent mit einem Oligonukleotid gekuppelt ist, das fähig ist, durch Hybridisierung mit einer Sequenz mit Spezifität für den DNA-Doppelstrang eine Tripelhelix zu bilden, wobei das Oligonukleotid eine Homopyrimidinsequenz, ausgewählt aus der Sequenz (CCT)ₙ, der Sequenz (CT)ₙ und der Sequenz (CTT)ₙ, worin n eine ganze Zahl zwischen 1 und 15 bedeutet, umfasst, **dadurch gekennzeichnet, dass** das Oligonukleotid einen Arm, bestehend aus einer geradkettigen Kohlenstoffkette (CH₂)ₙ, in der n eine ganze Zahl zwischen 1 und 18 bedeutet, und einem Amin, das fähig ist, mit dem Träger eine kovalente Bindung zu bilden, und einen Spacer, bestehend aus Basen, die die Hybridisierung nicht stören, enthält, wobei der Spacer mit der Homopyrimidinsequenz und dem Arm verbunden ist und der Arm an den Träger gebunden ist.

2. Träger nach Anspruch 1, **dadurch gekennzeichnet, dass** das Oligonukleotid methylierte Cytosine umfasst.

3. Träger nach einem der vorhergehenden Ansprüche, **dadurch gekennzeichnet, dass** es sich bei dem Träger um einen funktionalisierten Chromatografieträger, einen aus funktionalisierten Kunststoffoberflächen bestehenden Träger oder einen aus funktionalisierten Latexkügelchen bestehenden Träger handelt.

4. Träger nach Anspruch 3, **dadurch gekennzeichnet, dass** es sich bei dem Träger um einen funktionalisierten Chromatografieträger, ausgewählt aus Agarose, Acrylamid, Dextran, Acrylamidderivaten, Dextranderivaten, Poly(styroldivinylbenzol), gepfropftem Siliciumdioxid und nicht gepfropftem Siliciumdioxid, handelt.

5. Träger nach einem der vorhergehenden Ansprüche, **dadurch gekennzeichnet, dass** das Oligonukleotid eine Sequenz, ausgewählt aus den Sequenzen SEQ ID NO: 1, SEQ ID NO: 3, SEQ ID NO: 12, SEQ ID NO: 18, SEQ ID NO: 19 und SEQ ID NO: 26, umfasst.

6. Verfahren zur Herstellung eines Trägers nach Anspruch 1, das darin besteht, dass man das Oligonukleotid kovalent auf den Träger kuppelt.

7. Verfahren nach Anspruch 6, **dadurch gekennzeichnet, dass** das Oligonukleotid methylierte Cytosine umfasst.

8. Verfahren nach einem der Ansprüche 6 oder 7, **dadurch gekennzeichnet, dass** das Oligonukleotid funktionalisiert wird.

9. Verfahren nach Anspruch 7, **dadurch gekennzeichnet, dass** das Oligonukleotid durch eine terminale Thiol-, Amin- oder Carboxylgruppe in 5'- oder in 3'-Stellung modifiziert wird.

10. Verfahren nach einem der Ansprüche 6 bis 9, **dadurch gekennzeichnet, dass** es sich bei dem Träger um einen funktionalisierten Chromatografieträger, einen aus funktionalisierten Kunststoffoberflächen bestehenden Träger oder einen aus funktionalisierten Latexkügelchen bestehenden Träger handelt.

11. Träger nach Anspruch 10, **dadurch gekennzeichnet, dass** es sich bei dem Träger um einen funktionalisierten Chromatografieträger, ausgewählt aus Agarose, Acrylamid, Dextran, Acrylamidderivaten, Dextranderivaten, Poly(styroldivinylbenzol), gepfropftem Siliciumdioxid und nicht gepfropftem Siliciumdioxid, handelt.

12. Verfahren nach einem der Ansprüche 10 oder 11, **dadurch gekennzeichnet, dass** der Träger Disulfid-, Maleimid-, Amin-, Carboxyl-, Ester-, Epoxid-, Cyanbromid- oder Aldehydfunktionen trägt.

13. Verfahren zur Herstellung nach einem der Ansprüche 6 bis 12, **dadurch gekennzeichnet, dass** das Oligonukleotid eine Sequenz, ausgewählt aus den Sequenzen SEQ ID NO: 1, SEQ ID NO: 3, SEQ ID NO: 12, SEQ ID NO: 18, SEQ ID NO: 19 und SEQ ID NO: 26, umfasst.

## Claims

1. Support for the purification of a double-stranded DNA, **characterized in that** the said support is covalently coupled to an oligonucleotide capable of forming by hybridization a triple helix with a specific sequence of the double-stranded DNA, the said oligonucleotide comprising a homopyrimidine sequence chosen from the sequence (CCT)ₙ, the sequence (CT)ₙ and the sequence (CTT)ₙ, in which n is an integer between 1 and 15, **characterized in that** the oligonucleotide contains an arm consisting of a linear carbon chain (CH₂)ₙ, where n is an integer between 1 and 18, and an amine capable of forming a covalent bond with the support, and a spacer composed of bases which do not interfere with the hybridization, the said spacer being linked to the homopyrimidine sequence and to the arm and the said arm being bound to the support.

2. Support according to Claim 1, **characterized in that** the oligonucleotide comprises methylated cytosines.

3. Support according to any one of the preceding claims, **characterized in that** the support is a functionalized chromatography support, a support consisting of functionalized plastic surfaces or a support consisting of functionalized latex beads.

4. Support according to Claim 3, **characterized in that** the support is a functionalized chromatography support chosen from agarose, acrylamide, Dextran, acrylamide derivatives, Dextran derivatives, poly(styrenedivinylbenzene), grafted silica and non-grafted silica.

5. Support according to any one of the preceding claims, **characterized in that** the oligonucleotide comprises a sequence chosen from the sequences SEQ ID NO: 1, SEQ ID NO: 3, SEQ ID NO: 12, SEQ ID NO: 18, SEQ ID NO: 19, and SEQ ID NO: 26.

6. Process for preparing a support according to Claim 1, consisting in covalently coupling the oligonucleotide with the said support.

7. Process according to Claim 6, **characterized in that** the oligonucleotide comprises methylated cytosines.

8. Process according to either one of Claims 6 and 7, **characterized in that** the oligonucleotide is functionalized.

9. Process according to Claim 7, **characterized in that** the oligonucleotide is modified by a thiol, amine or carboxyl terminal group at the 5' or 3' position.

10. Process according to any one of Claims 6 to 9, **characterized in that** the support is a functionalized chromatography support, a support consisting of functionalized plastic surfaces or functionalized latex beads.

11. Process according to Claim 10, **characterized in that** the support is a functionalized chromatography support chosen from agarose, acrylamide, Dextran, acrylamide derivatives, Dextran derivatives, poly(styrenedivinylbenzene), grafted silica and non-grafted silica.

12. Process according to one of Claims 10 or 11, **characterized in that** the support carries disulphide, maleimide, amine, carboxyl, ester, epoxide, cyanogen bromide or aldehyde functional groups.

13. Process of preparation according to any one of Claims 6 to 12, **characterized in that** the said oligonucleotide comprises a sequence chosen from the sequences SEQ ID NO: 1, SEQ ID NO: 3, SEQ ID NO: 12, SEQ ID NO: 18, SEQ ID NO: 19, and SEQ ID NO: 26.


### LISTE DE SEQUENCES

(1) INFORMATIONS GENERALES:
   (i) DEPOSANT:
      (A) NOM: AVENTIS PHARMA S.A.
      (B) RUE: 20 AVENUE RAYMOND ARON
      (C) VILLE: ANTONY CEDEX
      (E) PAYS: FRANCE
      (F) CODE POSTAL: 92165
      (G) TELEPHONE: 40.91.69.22
      (H) TELECOPIE: 40.91.72.91
   (ii) TITRE DE L'INVENTION: PURIFICATION D'ADN PAR FORMATION DE TRIPLE HELICE AVEC UN OLIGONUCLEOTIDE IMMOBILISE
   (iii) NOMBRE DE SEQUENCES:25
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 25 paires de bases (B) TYPE: nucléotide (C) NOMBRE DE BRINS: simple (D) CONFIGURATION: linéaire
   (ii) TYPE DE MOLECULE: ADNc
   (xi) DESCRIPTION DE LA SEQUENCE: SEQ ID NO: 1:
      GAGGCTTCTT CTTCTTCTTC TTCTT 25
(2) INFORMATIONS POUR LA SEQ ID NO: 2:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
      CTTCCCGAAG GGAGAAAGG 19
(2) INFORMATIONS POUR LA SEQ ID NO: 3:
   (i) CARACTERISTIQUES DE LA SEQUENCE:
      (A) LONGUEUR: 21 paires de bases
      (B) TYPE: nucléotide
      (C) NOMBRE DE BRINS: simple (D) CONFIGURATION: linéaire
   (ii) TYPE DE MOLECULE: ADNc
   (xi) DESCRIPTION DE LA SEQUENCE: SEQ ID NO: 3:
      GAAGGGTTCT TCCCTCTTTC C 21
(2) INFORMATIONS POUR LA SEQ ID NO: 4:
   (i) CARACTERISTIQUES DE LA SEQUENCE:
      (A) LONGUEUR: 13 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
      GAAAAAGGAA GAG 13
(2) INFORMATIONS POUR LA SEQ ID NO: 5:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
      AAGGGAGGGA GGAGAGGAA 19
(2) INFORMATIONS POUR LA SEQ ID NO: 6:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 6:
      AAGGAGAGGA GGGAGGGAA 19
(2) INFORMATIONS POUR LA SEQ ID NO: 7:
   (i) CARACTERISTIQUES DE LA SEQUENCE:
      (A) LONGUEUR: 19 paires de bases
      (B) TYPE: nucléotide
      (C) NOMBRE DE'BRINS: simple
      (D) CONFIGURATION: linéaire
   (ii) TYPE DE MOLECULE: ADNc
   (xi) DESCRIPTION DE LA SEQUENCE: SEQ ID NO: 7:
      TTGGTGTGGT GGGTGGGTT 19
(2) INFORMATIONS POUR LA SEQ ID NO: 8:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 8:
      AAAAAAGGGA ATAAGGG 17
(2) INFORMATIONS POUR LA SEQ ID NO: 9:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 9:
      GATCCGAAGA AGAAGAAGAA GAAGAAGAAG AAGAAGAAGA AGAAGAAGAA GAAGAAGG 58
(2) INFORMATIONS POUR LA SEQ ID NO: 10:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 10:
      AATTCCTTCT TCTTCTTCTT CTTCTTCTTC TTCTTCTTCT TCTTCTTCTT CTTCTTCG 58
(2) INFORMATIONS POUR LA SEQ ID NO: 11:
   (i) CARACTERISTIQUES DE LA SEQUENCE:
      (A) LONGUEUR: 17 paires.de bases
      (B) TYPE: nucléotide
      (C) NOMBRE DE BRINS: simple
      (D) CONFIGURATION: linéaire
      (ii) TYPE DE MOLECULE: ADNc
   (xi) DESCRIPTION DE LA SEQUENCE: SEQ ID NO: 11:
      TGACCGGCAG CAAAATG 17
(2) INFORMATIONS POUR LA SEQ ID NO: 12:
   (i) CARACTERISTIQUES DE LA SEQUENCE:
      (A) LONGUEUR: 17 paires de bases
      (B) TYPE: nucléotide
      (C) NOMBRE DE BRINS: simple
      (D) CONFIGURATION: linéaire
   (ii) TYPE DE MOLECULE: ADNc
   (ix) FEATURE:
      (D) OTHER INFORMATION: toute les cytosines C de la séquence sont méthylée
   (xi) DESCRIPTION DE LA SEQUENCE: SEQ ID NO: 12:
      GAGGCTTCTT CTTCTTCTTC TTCTT 25
(2) INFORMATIONS POUR LA SEQ ID NO: 13:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 13:
      GATCCGAGAG AGAGAGAGAG AGAGAGAGAG AGAGAGAGAG AGAGAGAGAG AGAGAGGG 58
(2) INFORMATIONS POUR LA SEQ ID NO: 14:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 14:
      AATTCCCTCT CTCTCTCTCT CTCTCTCTCT CTCTCTCTCT CTCTCTCTCT CTCTCTCG 58
(2) INFORMATIONS POUR LA SEQ ID NO: 15:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 15:
      GATCCGGAGG AGGAGGAGGA GGAGGAGGAG GAGGAGGAGG AGGAGGAGGA GGAGGAGG 58
(2) INFORMATIONS POUR LA SEQ ID NO: 16:
   (i) CARACTERISTIQUES DE LA SEQUENCE:
      (A) LONGUEUR: 58 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 16:
      AATTCCTCCT CCTCCTCCTC CTCCTCCTCC TCCTCCTCCT CCTCCTCCTC CTCCTCCG 58
(2) INFORMATIONS POUR LA SEQ ID NO: 17:
   (i) CARACTERISTIQUES DE LA SEQUENCE:
      (A) LONGUEUR: 50 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 17:
      GGAGAGGAGG AGGAGGAGGA GGAGGAGGAG GAGGAGGAGG AGGAGGAGGA 50
(2) INFORMATIONS POUR LA SEQ ID NO: 18:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 18:
      AATGCCTCCT CCTCCTCCTC CTCCT 25
(2) INFORMATIONS POUR LA SEQ ID NO: 19:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 19:
      AGTGCTCTCT CTCTCTCTCT CTCTCT 26
(2) INFORMATIONS POUR LA SEQ ID NO: 20:
   (i) CARACTERISTIQUES DE LA SEQUENCE:
      (A) LONGUEUR: 66 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 20:
   GATCTGAAGA AGAAGAAGAA GAAGAAGAAG AAGAAGAAGA AGAAGAAGAA GAAGAACTGC 60
   AGATCT 66
(2) INFORMATIONS POUR LA SEQ ID NO: 21
   (i) CARACTERISTIQUES DE LA SEQUENCE:
      (A) LONGUEUR: 66 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 21:
   GATCAGATCT GCAGTTCTTC TTCTTCTTCT TCTTCTTCTT CTTCTTCTTC TTCTTCTTCT 60
   TCTTCA 66
(2) INFORMATIONS POUR LA SEQ ID NO: 22
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 22:
      ACAGTCATAA GTGCGGCGAC G 21
(2) INFORMATIONS POUR LA SEQ ID NO: 23
   (i) CARACTERISTIQUES DE LA SEQUENCE:
      (A) LONGUEUR: 39 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 23:
      GAAGAAGAGG AAGAAGAAGA AGAAGAAGAA GGAAGAGAA 39
(2) INFORMATIONS POUR LA SEQ ID NO: 24
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 24:
      CCGAATTCTG GGGACCAAAG CAGTTTC 27
(2) INFORMATIONS POUR LA SEQ ID NO: 25
   (i) CARACTERISTIQUES DE LA SEQUENCE:
      (A) LONGUEUR: 27 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 25:
      CCAAGCTTCA CTGTTCACGA CGGGTGT 27